# EUROPÄISCHE PATENTANMELDUNG

(11) **EP 2 067 484 A2**
(43) Veröffentlichungstag der Anmeldung: **10.06.2009**
(21) Anmeldenummer: 07834992.5
(22) Anmeldetag: 11.09.2007
(51) Int. Cl.: A61K 38/28, A61K 47/48, A61K 41/00

(54) **VERFAHREN ZUR HERSTELLUNG VON INSULIN IN FORM EINER ORALEN ZUBEREITUNG**

(30) Priorität: 13.09.2006 RU 2006132763
(71) Anmelder: "Concern O3" Company Limited, Moscow 117393 (RU)
(72) Erfinder: ARTAMONOV, Andrei Vladimirovich, Novosibirsk, 630005 (RU); RODIONOV, Petr Ivanovich, Moskovskaya oblast, 145033 (RU)
(74) Vertreter: Jeck, Anton
(86) Internationale Anmeldenummer: PCT/RU2007/000483
(87) Internationale Veröffentlichungsnummer: WO 2008/033058

(57) **Zusammenfassung**

Die Erfindung bezieht sich auf ein Verfahren zur Herstellung eines Insulinpräparates für orale Anwendung. Ist vorgesehen, dass es die Vermischung des Insulinpräparates mit wasserlöslichem Polymer einschließt, welches vorher durch eine Bestrahlung mit einer ionisierenden Strahlung aktiviert wird, dann wird das Verfahren billig durchführbar und seine therapeutische Effektivität wird wesentlich erhöht.

## Beschreibung

Die Erfindung bezieht sich auf ein Verfahren zur Herstellung eines Insulin-Präparates für orale Anwendung.

Die Erfindung gehört zum Gebiet der Pharmakologie und der Medizin, insbesondere zur Endokrinologie.

Insulin ist ein Polypeptidhormon mit Molekulargewicht von ca. 6000. Es beeinflusst auf alle Arten den Stoffwechsel im Körper: Es erhöht die Durchdringung von Glukose in das Körpergewebe und deren Nutzung im Gewebe, verringert den Gehalt an Glykogen in der Leber und erhöht seinen Anteil in den Muskeln, und somit die Intensität der Eiweisssynthese usw.

Hauptsächlich wird Insulin in den Menschenkörper durch subkutane oder intramuskuläre Injektionen des Präparates eingeleitet. Die Versuche der Einleitung des Insulins für die Kranken durch die am meisten physiologische und bequeme orale Weise (über den Mund) haben sich als erfolglos erwiesen, da das Insulin von den Verdauungsenzymen mit dem Verlust der Aktivität leicht hydrolisierbar ist.

Die Vorteile des Oralinsulins im Vergleich zu kommerziellen Injektionsformen sind offensichtlich, da die langwierigen täglichen Injektionen verschiedene ernste Komplikationen herbeirufen können. Sie werden von Schmerzsyndromen begleitet und führen zur Entwicklung von Lipodystrophie, welche nicht nur einen kosmetischen Fehler darstellt, sondern auch das Bedürfnis nach der Vergrösserung der Hormondosis hervorrufen kann. Außerdem können sie den geistigen Zustand, besonders bei Kindern, verletzen und rufen Stresszustände hervor, die zu einer noch mehr geäußerten Hyperglykämie führen und seinerseits den Bedarf an Hormon steigern kann.

Es ist ein Insulinpräparat für orale Anwendung bekannt, welches eine Wasser-Fett-Mikroemulsion darstellt, die aus Insulin, Lipiden und Proteaseninhibitor besteht. Die Mikroemulsion wird dann durch die Karboxymethylzellulose (Y.W.Cho, M. Flynm, Lancet, 1989, 30, p. 1518) gedeckt.

Ein wesentlicher Mangel dieses Präparates, neben der arbeitsintensiven und kostspieligen Technologie der Herstellung, ist die Nutzung der Karboxymethylzellulose als Träger, die für die Einwirkung der Mikroben, besonders unter den Bedingungen der Industrieproduktion, anfällig ist. Außerdem ist die Zellulose zum Sorbieren der bedeutenden Eiweißmengen fähig, die vom Puffer mit einer hohen lonenkraft abgewaschen werden muss. Die Durchführung einer solchen Prozedur in großen Maßstäben ist kostspielig und kann zu einer bedeutenden Inaktivierung des Insulins führen.

Es ist ein Verfahren zur Herstellung des Insulinpräparates für orale Anwendung auf dem Weg der Inkubation des Insulins mit Erythrozyten bekannt. Das Erythrozyt wird im Verhältnis 1-4:100 in Anwesenheit eines mehrfunktionalen verbindenden Agenten mit einer Endkonzentration von 0,15 - 0,25 % gewonnen. Dabei werden als Träger gewöhnliche Erythrozyte verwendet, die aus dem Rindviehblut, aus dem Schweinblut oder aus dem Menschenblut ausgesondert werden. Als Bindeagent wird vorzugsweise bromhaltiges Zyan, Zyanurchlorid oder Glutardialdehyd (Patent der Russischen Föderation, Nr. 2058788, KI. A 61 K 38/28, Verz. 27.04.96) verwendet. Vor der Nutzung wird das Präparat im Wasser emulsioniert.

Zum Nachteil des bekannten Verfahrens gehört eine hohe Giftigkeit der Bindeagenten und die damit verbundene Notwendigkeit einer kostspieligen Reinigung des Finalproduktes.

Es ist ein Verfahren zur Herstellung des Insulinpräparates als ein Gel für die orale Anwendung durch Immobilisierung des Insulins im Umfang des vernetzten Polymers bekannt. Das Polymer wird durch den Inhibitor eines proteolytischen Enzyms modifiziert (R. Z. Creenley, et. all Polymer Matrices for orol delivery, Polymer Preprints 1990, V.31, N 2, p. 182 - 183). Als vernetztes Polymer kommt Akryl- oder Methakrylsäure zur Anwendung, die mit Triathylenglykol-Di (Meth)-Akrylat vernetzt ist und als Inhibitor der proteolytischen Enzyme ist das Aprothenin als pankreatische Trypsininhibitor verwendet.

Zum Nachteil dieses Verfahrens gehört eine nicht hohe Widerstandsfähigkeit des hergestellten Präparates gegen Wirkung der Verdauungsenzyme. Die Folge ist eine niedrige Aktivität des ins Blut durchdringenden Insulins.

Am nächsten kommt dem anzumeldenden Verfahren ein Verfahren zur Herstellung des Insulinpräparates als ein Gel mittels Immobilisierung des Insulins im Umfang des vernetzten Polymers, welches durch den Inhibitor der proteolytischen Enzyme modifiziert ist. Als Inhibitor wird Ovomukoid aus dem Eiweiss in der Konzentration von 0,2 - 25 mg/g (des im Wasser gequellten Hydrogels) verwendet. Die Immobilisierung wird durch das Eintauchen des vernetzten modifizierten Polymers in die Wasserlösung des Insulins mit einer Konzentration von 0,01 - 5 mg/ml für 1-2 Stunden bis zu dem vollen Anschwellen des Polymers durchgeführt. Das abgeänderte Polymer wird in der Höhe von 0,01 - 1,0 g für 1 ml der Insulinlösung verwendet (Patent der Russischen Föderation Nr. 2066551, KI. A 61 K 38/28, Verz. 20.09.96).

Zum Nachteil dieses Verfahrens gehört die technologische Schwierigkeit zur Aussonderung des Ovomukoids sowie zur Herstellung des vernetzten Polymers, welches damit modifiziert wurde, um die Teuerung sowie die niedrige therapeutische Effektivität des hergestellten Präparates zu vermeiden.

Ausgehend von diesem Stand der Technik liegt der Erfindung die Aufgabe zugrunde, Verfahren zur Herstellung eines Insulinpräparates für orale Anwendung zu schaffen, das billig durchführbar ist und eine erhöhte therapeutische Effektivität mittels Immobilisierung des Insulins am wasserlöslichen Polymer aufweist.

Die gestellte Aufgabe wird durch die Merkmale des Anspruchs 1 gelöst.

Weitere zweckmäßige und vorteilhafte Maßnahmen der Erfindung gehen aus den Unteransprüchen hervor.

Ein wesentlicher Unterschied des neuen Verfahrens im Vergleich zum bekannten Verfahren ist, dass das Insulin vom Polymer modifiziert wird, das durch die ionisierende Strahlung aktiviert wurde. Dies bringt die Vereinfachung des Verfahrens und die Steigerung der therapeutischen Effektivität des Präparates. Bei der Bestrahlung während der strahlungschemischen Oxydierung im Polymer werden hochaktive Karbonylgruppen gebildet. Das auf diese Weise aktivierte Polymer bildet mit dem Insulin einen wasserlöslichen Komplex, der das Glukoseniveau bei der oralen Einleitung wirksam verringert. Infolge der hohen Lösbarkeit in Wasserlösungen dringt der Insulin-Polymer-Komplex ohne Diffusionsbeschränkungen vollständig in das Blut ein. Die Beispiele der Polymere schließen in sich folgendes ein: (werden aber darauf nicht beschränkt) Dextrane, Polyvinylpyrrolidon, Isoprenole, Polyakrylamid und Polyurethan.

### Das Verfahren wird in folgender Weise angewendet:

Man bereitet eine 1 - 50 % Polyäthylenoxid-Wasserlösung mit einem Molekulargewicht von 0,4 bis zu 40 kDa. Dann wird die Lösung mit einer hoch-energetischen ionisierenden Strahlung, vorzugsweise mit einer Gammastrahlung oder mit einem Strom von beschleunigten Elektronen in einer Dosis, die das Durchfließen der freiradikalen Reaktionen gewährleistet, von vorzugsweise 1,0- 5,0 Mrad, bestrahlt. In die Lösung des strahlungsaktivierten Polyäthylenoxids wird das Insulin bis zur Endkonzentration (nach dem Eiweiß) von 1 bis 10 mg/ml (oder nach der Aktivität des Insulins von 10 - 100 ME/ml entsprechend), im Verhältnis des Polyäthylenoxid: Insulin 1 - 500 (:1), eingeführt. Die Mischung wird dann im Laufe von 10 - 30 Minuten bis zum Darstellen einer gleichartig durchsichtigen oder leicht opaleszenten Lösung vermischt.

Die Nutzung des Ovomukoids als Proteaseninhibitor im bekannten Verfahren ermöglicht den Schutz des Insulins vor den proteolytischen Enzymen. Das Polyakrylamid-Gel übernimmt die Funktion der Deponierung des modifizierten Insulins. Bei der teilweisen Durchdringung in das Blut durch die Wand des Magen-Darm-Kanals wird das Insulin zielgerichtet in der Leber nicht deponiert, da es über keine Verwandtschaft zu den Organen des retikuloendothelialen Systems verfügt. In diesem Zusammenhang wird seine Wirkung ähnlich der Wirkung des Insulins bei der parenteralen Einleitung sein. Hingegen zeigt das Insulin, das durch ein strahlungsaktivierten Polymer modifiziert ist, die Eigenschaften des Basalinsulins, das heißt seine pharmakologische Wirkung ist dem physiologischen Mechanismus maximal angenähert. Dazu trägt das Polymer bei, das über die Fähigkeit verfügt, durch die Leberzellen ergriffen zu werden.

Das erzielte technische Ergebnis war aus den bekannten wissenschaftlichtechnischen Daten über die Eigenschaften der erwähnten Polymere und des Insulins nicht offensichtlich, da als Ergebnis der Modifikation des Insulins durch das strahlungsaktivierte Polymer der volle Verlust der spezifischen hypoglykämischen Aktivität des Insulins auf Kosten der Veränderung seiner Konformation sowie der Wechselwirkung mit Zelleninsulinrezeptor geschieht. Das neue Verfahren erlaubt nicht nur die spezifisch hypoglykämische Aktivität des Präparates des Insulins zu erhalten, sondern gewährleistet auch den maximalen physiologischen Mechanismus seiner Wirkung. Dies geschieht auf Kosten dessen, dass der aktivierte Polymerträger mit dem Insulin chemisch labile Bindungen eingeht und nur als ein Transportträger des Insulins in den Leberzellen verwendet wird, wo die Freisetzung des nativen Insulins stattfindet.

Die Erfindung wird an den nachfolgenden Beispielen einer konkreten Herstellung des Insulinpräparates veranschaulicht.

### Beispiel 1

Eine 10 % Polyäthylenoxid-Wasserlösung mit einem Molekulargewicht von 1,5 kDa wird mit einem Strom von beschleunigten Elektronen mit einer Dosis von 5,0 Mrad bestrahlt. In die bestrahlte Lösung wird das Insulin bis zur Endkonzentration von 10 mg in 1 ml (das Verhältnis Polyäthylenoxid : Insulin ist gleich 10 : 1) eingebracht. Die Mischung wird 10 Minuten lang vermischt und dann wird das Insulinpräparat als eine leicht opaleszente Lösung dargestellt. Der Ausstoß des fertigen Produktes beträgt 98%.

### Beispiel 2

Eine 50,0 % Polyäthylenoxid-Wasserlösung mit einem Molekulargewicht von 0,4 kDa wird mit einer Bremsgammastrahlung mit einer Dosis von 1,0 Mrad bestrahlt. In die bestrahlte Lösung wird das Insulin bis zur Endkonzentration von 1 mg in 1 ml (das Verhältnis Polyäthylenoxid : Insulin ist gleich 500 : 1) eingebracht. Die Mischung wird 30 Minuten lang vermischt und dann wird das Insulinpräparat als eine durchsichtige Lösung dargestellt. Der Ausstoß des fertigen Produktes beträgt 97 %.

### Beispiel 3

Eine 5 % Polyäthylenoxid-Wasserlösung mit einem Molekulargewicht von 15 kDa wird mit einem Strom der beschleunigten Elektronen mit einer Dosis von 2,5 Mrad bestrahlt. In die bestrahlte Lösung wird das Insulin bis zur Endkonzentration von 10 mg in 1 ml (das Verhältnis Polyäthylenoxid : Insulin ist gleich 5 : 1) eingebracht. Die Mischung wird 15 Minuten lang vermischt und dann wird das Insulinpräparat als eine leicht opaleszente Lösung dargestellt. Der Ausstoß des fertigen Produktes beträgt 99 %.

In der Tabelle 1 sind die Ergebnisse der Forschung der hypoglykämischen Wirkung des Insulin-Komplexes mit strahlungsaktiviertem Polyäthylenoxid auf die intakten Ratten der Wistar-Linie zusammengeführt. In der Versuchsgruppe wurde den Versuchstieren in den Magen einmalig je 1 ml des mit dem Polyäthylenoxid modifizierten Schweineinsulins (Zusammensetzung einmalig eingeführt: 30 ME/ml des Schweineinsulins, 12,5 mass % des strahlungsaktivierten Polyäthylenoxids 1500, das Verhältnis Polyäthylenoxid: Insulin ist 125 : 1) eingeführt. In der Kontrollgruppe wurde den Tieren in den Magen einmalig je 1 ml Schweineinsulin mit der Aktivität von 30 ME/ml eingeführt.

**Tabelle 1**

| Nr. des Tieres (Versuch) | Dauer der Messung des Glukoseniveaus im Blut, Min | | | | | | | | | | |
|---|---|---|---|---|---|---|---|---|---|---|---|
| | 0 | 60 | 120 | 180 | 240 | 300 | 360 | 420 | 480 | 540 | 600 |
| | Glukosenkonzentration im Blut, mmol/l | | | | | | | | | | |
| 1 | 7,9 | 5,1 | 5,6 | 5,3 | 5,1 | 5,1 | 4,6 | 3,6 | 5,4 | 5,7 | 5,9 |
| 2 | 5,9 | 5,3 | 5,0 | 4,8 | 4,3 | 4,7 | 4,9 | 4,4 | 4,9 | 6,1 | 5,9 |
| 3 | 5,2 | 5,2 | 6,3 | 5,1 | 4,7 | 4,6 | 4.3 | 4,3 | 5,2 | 6,1 | 4,9 |
| 4 | 7,1 | 7,1 | 6,2 | 5,7 | 4,9 | 4,6 | 4,7 | 4,9 | 5,9 | 4,8 | 4,6 |
| 5 | 6,1 | 6,0 | 6,7 | 6,0 | 6,3 | 6,2 | 4,7 | 5,6 | 6,3 | 5,6 | 5,1 |
| | | | | | | | | | | | |

| Nr. des Tieres (Kontrolle) | Dauer der Messung des Glukoseniveaus im Blut, Min | | | | | | | | | | |
|---|---|---|---|---|---|---|---|---|---|---|---|
| | 0 | 60 | 120 | 180 | 240 | 300 | 360 | 420 | 480 | 540 | 600 |
| | Glukosenkonzentration im Blut, mmol/l | | | | | | | | | | |
| 1 | 6,5 | 5,3 | 7,3 | 5,6 | 5,6 | 5,4 | 5,2 | 5,0 | 6,6 | 6,1 | 5,6 |
| 2 | 6,8 | 6,2 | 7,4 | 6,4 | 6,1 | 5,9 | 5,9 | 5,3 | 5,6 | 5,4 | 5,1 |
| 3 | 5,3 | 5,8 | 5,5 | 5,7 | 5,1 | 5,3 | 6,2 | 5,4 | 4,8 | 5,5 | 5,4 |
| 4 | 7,5 | 6,7 | 7,7 | 6,8 | 5,5 | 5,4 | 6,3 | 7,6 | 6,4 | 6,0 | 5,2 |
| 5 | 5,9 | 6,9 | 6,1 | 6,2 | 6,7 | 6,1 | 5,5 | 5,2 | 6,1 | 5,8 | 5,8 |
| Durchschnittswert (Versuch) | 6,44 | 5.74 | 5,96 | 5,38 | 5,06 | 5,04 | 4,64 | 4.56 | 5,54 | 5,66 | 5,28 |
| Durchschnittswert (Kontrolle) | 6,40 | 6,18 | 6,80 | 6,14 | 5,80 | 5,62 | 5,82 | 5,70 | 5,90 | 5,76 | 5,42 |

Aus den in der Tabelle 1 aufgeführten Ergebnissen ist ersichtlich, dass das durch das Polyäthylenoxid modifizierte Insulin die hypoglykämische Hauptaktivität erst 3 Stunden nach der Einleitung in den Magen vorzuzeigen beginnt und behält diese im Laufe von bis zu 10 Stunden. Die erhaltenen Daten zeugen von der Annäherung der Wirkung des modifizierten Insulins zur basalen Sekretion des Insulins durch die Bauchspeicheldrüse.

In der Tabelle 2 sind die Daten zur Untersuchung der hypoglykämischen Aktivität des durch das Polyäthylenoxid modifizierten gentechnischen Menscheninsulins anhand des Modells vom Alloxandiabetes bei den Ratten zusammengeführt. In der Versuchsgruppe wurde den Versuchstieren einmalig intrakutan je 1 ml des durch das Polyäthylenoxid modifizierten Insulins eingeführt (Zusammensetzung: 50 ME/ml des menschlichen Insulins, 12,5 mass. % des strahlungsaktivierten Polyäthylenoxids 1500, das Verhältnis Polyäthylenoxid: Insulin ist 70: 1). In der Kontrollgruppe wurde den Tieren je 1 ml des menschlichen Insulins mit der Aktivität von 50 ME/ml eingeführt.

**Tabelle 2**

| Versuchsserie | Dauer der Messung des Glukoseniveaus im Blut, Stunden | | | | | | |
|---|---|---|---|---|---|---|---|
| | Ausgangswert | 2 | 4 | 6 | 8 | 10 | 24 |
| | | Änderung der Glukosenkonzentration im Blut, mmol/l | | | | | |
| Kontrolle (n = 5) | 23,4±2,8 | -1,1±2,1 | -1,2±1,7 | -5,1±2,3 | -0,4±4.8 | +1,3±3.8 | -0.8±1,8 |
| Versuch (n =5) | 26,2±3,5 | -5.8±1.0 | -9,1±1,0 | -8.9±1,5 | -4,9±3,2 | -0,5±2.3 | -4,1±2.2 |

Wie es aus den vorgestellten Ergebnissen ersichtlich ist, verfügt das modifizierte Insulin über eine ausgeprägte hypoglykämische Aktivität bei der Einführung in den Magen von Ratten mit einem Alloxanmodell von Diabetes.

In der Tabelle 3 sind die vergleichenden Daten zum Einfluss des durch das Polyathylenoxid modifizierten Insulins (das Verhältnis Polyäthylenoxid: Insulin ist gleich 70 : 1) auf die absoluten Bedeutungen des Insulinpegels im Blutplasma bei den Ratten bei der einmaligen Einleitung zusammengeführt. In der Versuchsgruppe wurde den intakten Ratten der Linie Wistar, Gewicht: 200 - 240 g das durch das Polyäthylenoxid modifizierte menschliche Insulin ausgehend aus der Berechnung: 250 ME/kg, eingeführt. In der Kontrollgruppe wurde den Tieren die äquivalente Menge des nicht modifizierten Insulins eingeführt. Der Insulingehalt im Blutplasma bei den Ratten (in µED/ml) wurde durch die Immuno-Enzym-Methode bestimmt.

**Tabelle 3**

| Versuchsserie | Ausgangsdaten | Dauer der Messung des Insulinpegels im Blutplasma bei den Ratten, Stunden | | |
|---|---|---|---|---|
| | | 4 | 5 | 6 |
| | | Insulinpegel im Blutplasma bei den Ratten (in µED/ml) | | |
| Intakte Tiere | 26±7 | 16±9 | 14±7 | 16±8 |
| Kontrolle | 27±10 | 10±3 | 15±6 | 17±10 |
| Versuch | 23±6 | 23±6 | 35±10 | 20±6 |

Aus den in der Tabelle zusammengeführten Daten ist es ersichtlich, dass das Insulin, das durch das Polyäthylenoxid modifiziert wurde, bei der Einleitung in den Magen entweder zur Erhöhung des Insulinpegels im Blutplasma führt oder seine Senkung verhindert, d.h. das modifizierte Insulin nähert sich maximal dem Basalinsulin an, welches von der Bauchspeicheldrüse im Rahmen der physiologischen Norm sezerniert wird.

Das neue Verfahren zur Herstellung eines Insulinpräparates für orale Anwendung zeichnet sich im Unterschied zum bekannten Verfahren durch die Einfachheit und die Wirtschaftlichkeit aus, da seine Herstellung nur aus zwei Phasen besteht, in welchen der Polymerträger - das Polyäthylenoxid - eingesetzt wird. Die technologisch einfache Weise seiner Aktivierung ist die Einwirkung der ionisierenden Strahlung auf die Polymerlösung, sowie die Modifikation des Insulins durch das aktivierte Polymer mittels der Einleitung des Insulins in die Lösung des aktivierten Polymers bis zur erforderlichen Konzentration (die Aktivität). Das Insulinpräparat, das durch das neue Verfahren hergestellt wird, verfügt über eine hohe therapeutische Aktivität, die sowohl nach der Senkung des Glukosenieavus im Blut, als auch nach der direkten Bestimmung des Insulinpegels im Blut gegeben ist.

## Patentansprüche

1. Verfahren zur Herstellung eines Insulinpräparates für orale Anwendung,
**dadurch gekennzeichnet,**
**dass** es die Vermischung des Insulinpräparates mit wasserlöslichem Polymer einschließt, welches vorher durch eine Bestrahlung mit einer ionisierenden Strahlung aktiviert wird.

2. Verfahren nach Anspruch 1,
**dadurch gekennzeichnet,**
**dass** als Polymer Polyethylenoxid mit einem Molekulargewicht von 0,4 - 40 kDA und einer Konzentration von 1,0 bis 50,0 % verwendet wird, wobei das Verhältnis Insulin-Polyethylenoxid 1: (1 bis 500) bis zur Endkonzentration des Insulins im Gemisch von 1 bis 10 mg/ml beträgt.

3. Verfahren nach Anspruch 1,
**dadurch gekennzeichnet,**
**dass** das Polyethylenoxid durch die Bestrahlung mit einem Strom von beschleunigten Elektronen oder durch eine Gamma-Strahlung mit einer Dosis von 1,0 bis 5,0 Mrad aktiviert wird.

4. Verfahren nach Anspruch 1,
**dadurch gekennzeichnet,**
**dass** eine 10 %ige Polyäthylenoxid-Wasser-Lösung mit einem Molekulargewicht von 1,5 kDa mit einem Strom von beschleunigten Elektronen mit einer Dosis von 5,0 Mrad bestrahlt wird,
**dass** in das Insulin bis zur Endkonzentration von 10 mg in 1 ml (das Verhältnis Polyäthylenoxid : Insulin ist gleich 10 : 1) eingebracht wird,
**dass** die Mischung 10 Minuten lang vermischt wird und
**dass** dann das Insulinpräparat als eine leicht opaleszente Lösung als fertiges Produkt mit 98 % ausgestoßen wird.

5. Verfahren nach Anspruch 1,
**dadurch gekennzeichnet,**
**dass** eine 50 %ige Polyäthylenoxid-Wasser-Lösung mit einem Molekulargewicht von 0,4 kDa mit einer Bremsgammastrahlung mit einer Dosis von 1 Mrad bestrahlt wird,
**dass** in die bestrahlte Lösung das Insulin bis zur Endkonzentration von 1 mg in 1 ml (das Verhältnis Polyäthylenoxid: Insulin ist gleich 500 : 1) eingebracht wird,
**dass** die Mischung 30 Minuten lang vermischt wird und
**dass** dann das Insulinpräparat als fertiges Produkt mit 97 % ausgestoßen wird.

6. Verfahren nach Anspruch 1,
**dadurch gekennzeichnet,**
**dass** eine 5 %ige Polyäthylenoxid-Wasserlösung mit einem Molekulargewicht von 15 kDa mit einem Strom von beschleunigten Elektronen mit einer Dosis von 2,5 Mrad bestrahlt wird,
**dass** in die bestrahlte Lösung das Insulin bis zur Endkonzentration von 10 mg : 1 ml (das Verhältnis Polyäthylenoxid : Insulin ist gleich 5 : 1) eingebracht wird,
**dass** die Mischung 15 Minuten lang vermischt wird und
**dass** dann das Insulinpräparat als eine leicht opaleszente Lösung als fertiges Produkt mit 99 % ausgestoßen wird.
